(19) Europäisches Patentamt
European Patent Office
Office européen des brevets

(11) Publication number : 0 467 707 A1

(12) EUROPEAN PATENT APPLICATION

(21) Application number : 91306610.6

(51) Int. Cl.$^5$ : C07D 231/16

(22) Date of filing : 19.07.91

(30) Priority : 20.07.90 US 555034

(43) Date of publication of application :
22.01.92 Bulletin 92/04

(84) Designated Contracting States :
AT BE CH DE DK ES FR GB GR IT LI LU NL SE

(71) Applicant : BUCKMAN LABORATORIES INTERNATIONAL, INC.
1256 North McLean Boulevard
Memphis Tennessee 38108 (US)

(72) Inventor : Fenyes, Joseph G.
1827 Oakhill Cove
Memphis, Tennessee 38138 (US)

(74) Representative : Matthews, Derek Peter et al
Frank B. Dehn & Co. Imperial House 15-19 Kingsway
London WC2B 6UZ (GB)

# (54) Process for the preparation of 1-Hydroxymethylpyrazoles.

(57) A process for the preparation of a 1-hydroxymethylpyrazole comprising combining a methylpyrazole with water and an aqueous formaldehyde solution to form a 1-hydroxymethylpyrazole in a mother liquor, filtering said 1-hydroxymethylpyrazole from said mother liquor, and combining said mother liquor with a second aqueous formaldehyde solution and methylpyrazole.

EP 0 467 707 A1

The subject of the present invention is a method of making 1-hydroxymethylpyrazoles in high yields and high purity.

1-hydroxymethylpyrazole compounds are known microbicides which can be used as preservatives for aqueous systems containing organic materials. U.S. Patent No. 4,801,362 discloses the use of these compounds in latexes, surfactants, dispersants, stabilizers, thickeners, adhesives, starches, waxes, proteins, emulsifying agents, detergents, cellulose products and resins formulated in aqueous solutions, emulsions, or suspensions. These 1-hydroxymethylpyrazoles can also be used to combat and control microorganisms, e.g. bacterial and fungal plant pathogens, as disclosed in European Patent application of Buckman Laboratories International, Inc. filed concurrently herewith.

Several processes are known in the art to make 1-hydroxymethylpyrazoles. For example, Dvoretzky et al., "Formaldehyde Condensation in the Pyrazole Series," 15 Journ. Org Chem. 1285-1288 (1952) describe the production of 3,5-dimethyl-1-hydroxymethylpyrazole by the treatment of 3,5-dimethylpyrazole with 35% formalin in a mixture of water and ethanol as solvent. After standing at 30°C for 42 hours, the mixture was extracted with several portions of chloroform and, after evaporating the combined extracts, the solid product had to be recrystallized in order to obtain the title compound in 71% yield.

J. Huttel et al., "Die Mannichsche Reaktion der Pyrazole," 85 Chemische Berichte 820-826 (1952) obtained 3,5-dimethyl-1-hydroxymethylpyrazole in less than 55% yield when they treated a methanolic solution of 3,5-dimethylpyrazole for three hours at room temperature with an aqueous formaldehyde solution. By extracting the mother liquor with ether, an additional 32% of 3,5-dimethyl-1-hydroxymethylpyrazole, or a total of 87.0% yield was obtained.

While these prior art processes have proven adequate, these prior art processes contain or require the use of ethanolic or methanolic solutions, and the products obtained from these prior art processes have to be recrystallized in order to obtain higher purities, which then results in lower yields. A process which overcomes these inherent disadvantages is desired.

The above discussed disadvantages are overcome by the present invention, which relates to a novel process for the preparation of a 1-hydroxymethylpyrazole of formula (I):

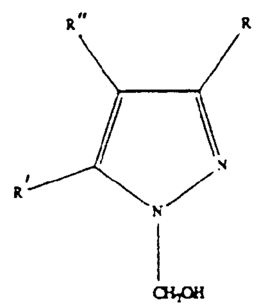

(I)

wherein R and R′ are independently hydrogen or alkyl of 1 to 4 carbon atoms, and R″ is hydrogen, a halogen atom, preferably F, Cl, Br or I, or a nitro group. The alkyl is preferably methyl.

The process comprises combining a methylpyrazole with water and an aqueous formaldehyde solution to form a 1-hydroxyethyl-pyrazole in a mother liquor, and then filtering the 1-hydroxy-methylpyrazole from the mother liquor. The mother liquor can be combined with a second aqueous formaldehyde solution and an aqueous methylpyrazole solution, and the entire above process repeated until the desired yield is obtained. The desired yield can be 95%-100%.

The aqueous formaldehyde solution can contain from about 30% to about 37% formaldehyde, by weight, preferably approximately 37% formaldehyde. The aqueous methylpyrazole solution can contain from about 40% to about 85% methylpyrazole, by weight, preferably approximately 60% methylpyrazole. The formaldehyde is preferably present in an excess from about 2 mole % to about 10 mole % relative to the methylpyrazole, and most preferably about 5 mole %.

The process can occur at a process temperature in the range of about 50°C to about 100°C, preferably in the range of about 80°C to about 90°C.

The present invention provides a process for the production of a 1-hydroxyethylpyrazole which requires no additional purification steps to obtain high yields with high purity.

Initially, the methylpyrazole is preferably combined with water to form a slurry. The slurry is reacted with a hydroxymethylating agent comprising 30-37%, preferably 37% by weight formaldehyde in aqueous solution.

The reaction temperature is maintained in the range of about 50° to about 100°C, preferably in the range of about 80°C to about 90°C. After the reaction is complete, the resulting mixture is allowed to cool, during which time it crystallizes and is then filtered.

Preferably, the mother liquor obtained during the above filtration is then replenished with additional aqueous formaldehyde solution, and the mixture is then used to prepare subsequent preparations of 1-hydroxyethylpyrazoles. These 1-hydroxyethylpyrazoles are in the range of about 95 to about 100% yield and having a purity greater than about 95% and typically about 100%. By reusing the mother liquors in subsequent preparations of 1-hydroxymethylpyrazoles, substantially theoretical yields of the product can be obtained.

Examples of 1-hydroxymethylpyrazoles which can be obtained by the process of the present invention include but are not limited to:

3,5-dimethyl-1-hydroxymethylpyrazole,
3,5-dimethyl-4-fluoro-1-hydroxymethylpyrazole,
3,5-dimethyl-4-bromo-1-hydroxymethylpyrazole,
3,5-dimethyl-4-iodo-1-hydroxymethylpyrazole,
3,5-dimethyl-4-chloro-1-hydroxymethylpyrazole, and
3,5-dimethyl-4-nitro-1-hydroxymethylpyrazole.

To illustrate the nature of the invention, the following example is given. It should be understood, however, that the invention is not to be limited to the specific conditions or details set forth in this example.

Example

Into a 1000 mL three-necked round-bottom flask were placed 126.1 g (1.31 moles) of 3,5-dimethylpyrazole and 250 mL of tap water. The resulting slurry was vigorously agitated and heated to give a clear solution (50-60°C). At this point, heating was discontinued and 111.5 g (1.37 moles) of a 37% aqueous solution of formaldehyde was added at such a rate as to keep the temperature below 90°C. A very exothermic reaction resulted. After completion of this addition, heating was continued to maintain a temperature between 80 and 90°C for 45 minutes to an hour. The mixture was allowed to cool to room temperature. Finally, the crystalline mass was chilled to about 10°C by means of an ice-water bath. The white crystalline mass was filtered by suction. The product weighed 152.0 g (92% of the theory) m.p. 112-114°C.

Subsequent preparations of 3,5-dimethyl-1-hydroxymethyl-pyrazole were obtained by recovering the aqueous mother liquor and adding a 37% aqueous solution of formaldehyde. The process was repeated as described above. Yields obtained were between 95 and 100%, and NMR gas chromatography revealed purity levels at 99.9%.

## Claims

**1.** A process for the preparation of a 1-hydroxymethyl-pyrazole of formula (I):

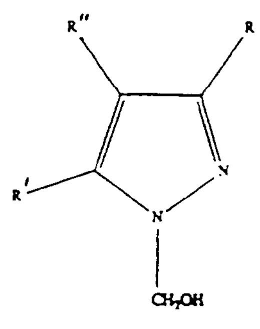

(I)

wherein R and R′ are independently hydrogen or alkyl of 1 to 4 carbon atoms, and R" is hydrogen, a halogen atom F, Cl, Br or 1, or a nitro group, said process comprising:

(a) combining an aqueous methylpyrazole solution and an aqueous formaldehyde solution to form a 1-hydroxymethylpyrazole in a mother liquor; and

(b) filtering said 1-hydroxymethylpyrazole from said mother liquor.

**2.** A process as claimed in claim 1 further comprising:

(c) combining said mother liquor with a second aqueous formaldehyde solution and a methylpyrazole

in aqueous solution; and
(d) repeating steps (b) and (c) until a desired yield is obtained.

3. A process as claimed in claim 2 wherein the yield is in the range of from 95 to 100%.

4. A process as claimed in claim 1 wherein said alkyl is methyl.

5. A process as claimed in claim 1 wherein said aqueous formaldehyde solution contains from 30% to 37% formaldehyde, by weight.

6. A process as claimed in claim 5 wherein said aqueous formaldehyde solution contains 37% formaldehyde, by weight.

7. A process as claimed in claim 1 wherein the temperature is maintained in the range of from 50°C to 100°C.

8. A process as claimed in claim 7 wherein the temperature is maintained in the range of from 80°C to 90°C.

9. A process as claimed in claim 1 wherein said aqueous methylpyrazole solution contains from 40% to 85% methylpyrazole, by weight.

10. A process as claimed in claim 9 wherein said aqueous methylpyrazole solution contains 60% methylpyrazole, by weight.

11. A process as claimed in claim 1 wherein said 1-hydroxymethylpyrazole is 3,5-dimethyl-1-hydroxymethylpyrazole, 3,5-dimethyl-4-fluoro-1-hydroxymethylpyrazole, 3,5-dimethyl-4-bromo-1-hydroxymethylpyrazole, 3,5-dimethyl-4-iodo-1-hydroxymethylpyrazole, 3,5-dimethyl-4-chloro-1-hydroxymethylpyrazole, or 3,5-dimethyl-4-nitro-1-hydroxymethylpyrazole.

European Patent Office

# EUROPEAN SEARCH REPORT

Application Number

EP 91 30 6610

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A | US-A-2 883 392 (ORTHO PHARMACEUTICAL CORPORATION ) | | C07D231/16 |
| A | TETRAHEDRON, (INCL. TETRAHEDRON REPORTS) vol. 45, no. 13, 1989, OXFORD GB pages 4253 - 4262; ALAN R. KATRITZKY,A.O.: 'Formaldehyde : A Reagent for Simultaneous Protection of Heterocyclic NH and Activation of Alternative Locations to Electrophilic Attack. Part II. A New Synthetic Method for the 5(3)-Substitution of N-Unsubstituted Pyrazoles.' | | |
| D,A | US-A-4 801 362 (BUCKMAN LABORATORIES INTERNATIONAL,INC.) | | |
| D,A | JOURNAL OF ORGANIC CHEMISTRY. vol. 15, no. 6, November 1950, EASTON US pages 1285 - 1288; ISAAC DVORETZKY,A.O.: 'Formaldehyde Condensation in the Pyrazole series' | | TECHNICAL FIELDS SEARCHED (Int. Cl.5 ) |
| D,A | CHEMISCHE BERICHTE. vol. 85, no. 7-8, 1952, WEINHEIM DE pages 820 - 826; RUDOLF HÜTTEL,U,A,: 'Die Mannichsche Reaktion der Pyrazole.' | | C07D |

The present search report has been drawn up for all claims

| Place of search | Date of completion of the search | Examiner |
|---|---|---|
| THE HAGUE | 08 NOVEMBER 1991 | DE BUYSER I.A.F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)